(19) **Europäisches Patentamt**

**European Patent Office**

**Office européen des brevets**

(11) **EP 1 339 335 B1**

(12) **EUROPÄISCHE PATENTSCHRIFT**

(45) Veröffentlichungstag und Bekanntmachung des Hinweises auf die Patenterteilung:
**18.08.2004   Patentblatt 2004/34**

(21) Anmeldenummer: **00979310.0**

(22) Anmeldetag: **08.12.2000**

(51) Int Cl.$^7$: **A61B 17/70**

(86) Internationale Anmeldenummer:
**PCT/CH2000/000654**

(87) Internationale Veröffentlichungsnummer:
**WO 2002/045606 (13.06.2002 Gazette 2002/24)**

(54) **VORRICHTUNG ZUR FIXATION VON KNOCHEN, INSBESONDERE VON WIRBELKÖRPERN RELATIV ZUEINANDER**

DEVICE FOR FIXING BONES, PARTICULARLY VERTEBRAL BODIES, IN RELATION TO ONE ANOTHER

DISPOSITIF POUR FIXER DES OS, NOTAMMENT DES CORPS VERTEBRAUX LES UNS PAR RAPPORT AUX AUTRES

(84) Benannte Vertragsstaaten:
**AT BE CH CY DE DK ES FI FR GB GR IE IT LI LU MC NL PT SE TR**
Benannte Erstreckungsstaaten:
**SI**

(43) Veröffentlichungstag der Anmeldung:
**03.09.2003   Patentblatt 2003/36**

(73) Patentinhaber: **SYNTHES AG Chur**
**7002 Chur (CH)**

(72) Erfinder:
• **SCHLÄPFER, Fridolin**
  **CH-8750 Glarus (CH)**
• **HESS, Martin**
  **CH-4434 Hölstein (CH)**
• **TAGWERKER, Konrad**
  **Jardim Bot., BR-13501-190 Rio Claro, SP (BR)**

(74) Vertreter: **Lusuardi, Werther Giovanni, Dr.**
**Dr. Lusuardi AG,**
**Kreuzbühlstrasse 8**
**8008 Zürich (CH)**

(56) Entgegenhaltungen:
WO-A-98/05263          WO-A-98/31293
US-A- 5 300 074        US-A- 5 501 684
US-A- 5 741 256

**Beschreibung**

Die Erfindung bezieht sich auf eine Vorrichtung zur Fixation von Knochen, insbesondere von Wirbelkörpern relativ zueinander, gemäss dem Oberbegriff des Patentanspruchs 1.

Instabilitiäten von Wirbelsäulen-Bewegungssegmenten, welche durch Wirbelfrakturen, degenerative Veränderungen, etc. verursacht sein können, bedingen oft eine Fusion der betroffenen Segmente. Um die für eine knöcherne Fusion notwendige Ruhigstellung der zu fusionierenden Bewegungssegmente zu erreichen, werden vielfach die entsprechenden Segmente mit einem Fixationssystem stabilisiert. Diese Fixationssysteme können entweder von posterior eingebracht und verankert werden, wobei die Verankerung mittels Knochenschrauben in den Pedikeln erfolgt, oder von anterior bzw. antero-lateral, wobei die Verankerung mittels Knochenschrauben in den Wirbelkörpern erfolgt.

Die Qualität der Verankerung der Fixationssysteme hängt stark von der Qualität der Knochenstrukturen ab. Dies gilt vor allem bei antero-lateral verankerten Fixationssystemen. Je grösser der Osteoporosegrad ist, desto grösser ist die Gefahr, dass die Knochenschrauben schon bei kleinen Lasten durch den Knochen schneiden. Verwendung von dicken Schrauben verkleinert das Risiko eines Durchschneidens. Andererseits möchte man aber doch nicht zu dicke Schrauben verwenden, um die Knochenstruktur im Wirbelkörper nicht zu stark zu zerstören.

Lösungen, um das Risiko eines Durchschneidens der Verankerungselemente zu reduzieren, werden in der DE 296 00 879 AESCULAP und in der WO 00/10473 AEBI aufgezeigt. Dabei geht es prinzipiell um eine Hohlschraube, die in den Wirbelkörper eingedreht wird. Die Hohlschraube hat den Vorteil, dass sie einen grossen Aussendurchmesser aufweist, ohne viel Knochen verdrängen zu müssen. Beim Eindrehen bleibt im Zentrum der Hohlschraube ein Knochenzapfen stehen. Nachteilig an dieser Lösung ist, dass

a) beim Eindrehen der Hohlschraube die vaskuläre Versorgung des im Zentrum verbleibenden Knochenzapfens grossenteils zerstört wird, was vor allem bei osteoporotischen Knochen zu einem Problem führen kann; und

b) bei Knochen mit noch funktionierenden Reparaturmechanismen die Hohlschraube so stark einwachsen kann, dass sie bei Revisionen schwierig zu entfernen ist, bzw. beim Entfernen ein grosser knöcherner Schaden entsteht (an der Halswirbelsäule eingesetzte Hohlschrauben zum Beispiel konnten zum Teil nicht mehr entfernt werden).

Im Zusammenhang mit der operativen Versorgung von Brüchen von Röhrenknochen wurden Techniken zur intramedullären Stabilisierung entwickelt, die abgewandelt auch bei der Wirbelsäule mit Erfolg eingesetzt werden könnten, um das Problem der Verankerung von anterioren und antero-lateralen Wirbelsäulen-Fixationssystemen zu lösen.

Marknägel werden zur Schienung von frakturierten Röhrenknochen eingesetzt, indem sie den proximalen Teil des gebrochenen Röhrenknochens mit dem distalen Teil intramedulär verbinden. Auf Grund seiner Geometrie kann der Marknagel aber nur kleine Rotations- und Axialbelastungen aufnehmen. Dies ist kein Problem, solange der gebrochene Knochen unter Axiallast in der Lage ist, die Höhe zu halten und die Fraktur mehr oder weniger diaphysär liegt. Sobald aber multifragmentäre Brüche vorliegen, muss der Marknagel proximal und distal verankert werden. Damit dient der Marknagel nicht mehr nur zur Schienung, sondern ähnlich wie bei der Wirbelsäule als ein proximal und distal verankerter Längsträger, der Kräfte und Momente in allen Ebenen von proximal nach distal übertragen kann. Die Verankerungsimplantate sind im Fall des Marknagels Schrauben, die distal und proximal quer durch den Knochen und den Marknagel geführt werden. Bei Osteoporose und bei gelenknahen Frakturen ist die Verankerung des Marknagels durch Schrauben nicht mehr genügend. Spiralartig verdrehte, klingenförmige Implantate wie sie in der US 3,433,220 ZICKEL, der EP 0 411 273 FRIGG, der US 4,103,683 NEUFELD, der US 4,978,349 FRIGG, der DE 43 18 150 FRIEDL, der EP 0 491 138 FRIGG (Basis für den Oberbegriff des Anspruchs 1.), der DE 37 30 570 MATTHECK, der WO 98/30164 BRESINA und der WO 99/45858 HUNGERBÜHLER beschrieben werden und wie sie auch klinisch eingesetzt werden, sind ungeeignet für den Einsatz an der Wirbelsäule.

Hier will die Erfindung Abhilfe schaffen. Der Erfindung liegt die Aufgabe zugrunde, eine Wirbelsäulenfixationsvorrichtung zu schaffen, welches mittels spiralartig verdrehten, klingenartigen Verankerungselementen an den Wirbelkörpern befestigbar ist und folgende zusätzliche Bedingungen berücksichtigt:

a) Setzen der Verankerungsimplantate vor dem Einbringen des Längsträgers, wodurch sich komplizierte Zielgeräte und ein spezifisches auf den Längsträger bezogenes Ausrichten der Verankerungselemente erübrigen;

b) der Längsträger wird auf das Ende des Verankerungsimplantates aufgesetzt;

c) Verbindungen zwischen Längsträger und Verankerungsimplantat, welche polyaxial, spielfrei, winkelstabil, blockierbar und wieder lösbar sind; und

d) Verankerungselemente sollen mit Transportvorrichtung einziehbar sein, wodurch sich ein unkontrolliertes Hämmern erübrigt.

Die Erfindung löst die gestellte Aufgabe mit einer Vorrichtung zur Fixation von Knochen, insbesondere von Wirbelkörpern relativ zueinander, welche die Merkmale des Anspruchs 1 aufweist.

Die erfindungsgemässe Vorrichtung zur Fixation von Knochen, insbesondere von Wirbelkörpern relativ zueinander umfasst im wesentlichen einen longitudinalen Längsträger mit einer Zentralachse und n Verankerungselemente ($2 \leq i \leq n$) mit Längsachsen, je einem vorderen Ende und je einem hinteren Ende. Die Längsachsen der Verankerungselemente lassen sich vorzugsweise in einem Winkel zwischen 65° und 115° zur Zentralachse des Längsträgers anordnen, während die Verankerungselemente an das hintere Ende angrenzend, gegen das vordere Ende hin vorzugsweise klingenförmig ausgebildet sind. Die vorzugsweise winkelvariierbare Verbindung der Verankerungselemente wird dadurch erreicht, dass jedes Verankerungselement am hinteren Ende Aufnahmemittel für den Längsträger mit vom hinteren Ende her bedienbaren Feststellmitteln zur lösbaren Arretierung der Verbindung zwischen Längsträger und Verankerungselement umfasst. Die arretierte Verbindung lässt keine Relativbewegungen zwischen Längsträger und Verankerungselement zu und nimmt Kräfte und Momente in allen drei Achsrichtungen eines dreidimensionalen Koordinatensystems auf.

Je nach Ausführungsform der Aufnahmemittel ist der Winkel zwischen den Längsachsen der Verankerungselemente und der Zentralachse des Längsträgers fest, um eine Achse variierbar oder in anderen Ausführungsformen der erfindungsgemässen Vorrichtung polyaxial einstellbar.

Die Oberfläche der Klinge kann ebenfalls auf einer durch die Länge L und die Breite B eingeschlossenen Seite oder auf beiden durch die Länge L und die Breite B eingeschlossenen Seiten verschiedenartig ausgestaltet sein:

a) glatte Oberfläche;
b) Sägezähne aufweisend;
c) Fischschuppenartig;
d) eine pfeilförmige Verzahnung aufweisend;
e) rauhe Oberfläche (z.B. geätzt, plasmabeschichtet, gestrahlt); oder
f) Löcher (biologische Verriegelung, indem der Knochen durch die Löcher wächst).

Dabei können die Oberflächenstrukturen einseitig, beidseitig oder nur partiell auf der Klingenoberfläche angebracht sein. Die unterschiedlichen Ausgestaltungen der Klinge sind mit den verschiedenen Oberflächen kombinierbar.

Das Verankerungselement wird mit Hilfe einer Transportvorrichtung in den Wirbelkörper eingebracht. Mögliche Transportsysteme sind:

a) Transportschraube ist in der Klinge (an der Spitze) integriert;

b) In der Gegenkortikalis verankertes Element (Schraube, Dübel, Haken etc. mit Verlängerung) dient als Widerlager, um die Klinge in den Knochen zu ziehen; oder
c) Vorrichtung wird in der Kortikalis verankert, um das Verankerungselement in den Knochen zu drükken.

Weitere vorteilhafte Ausgestaltungen der Erfindung sind in den abhängigen Ansprüchen gekennzeichnet.

Die Erfindung bietet gegenüber den über Schrauben mit den Wirbeln verankerten Vorrichtungen folgende Vorteile:

1) Der Widerstand bezüglich Durchschneiden im Knochen ist erhöht bei gleichzeitiger Reduktion der verdrängten Knochenmasse:

Gleiche tragende Fläche pro Längeneinheit:

- Implantat mit 2 Knochenschrauben pro Wirbel mit Kerndurchmesser d = 5 mm:
Tragende Fläche pro Flächeneinheit : $2$ x d x Länge / Länge = $10 \text{ mm}^2$ / mm
Verdrängung pro Längeneinheit: $2$ x $d^2$ x Pi/4 x Länge / Länge = $39 \text{ mm}^2$ / mm

- Implantat mit klingenförmigem Verankerungselement pro Wirbel mit Breite B = 10 mm, Seele in der Mitte mit Durchmesser d = 5 mm und Klingendicke D = 1,2 mm:
Tragende Fläche pro Flächeneinheit : $2$ x d x Länge / Länge = $10 \text{ mm}^2$ / mm
Verdrängung pro Längeneinheit: ((B-d) x H + $d^2$ x Pi/4) x Länge / Länge = $25,6 \text{ mm}^2$ / mm

D.h. es resultiert eine Reduktion der Verdrängung (exkl. Gewinde) um 34% bei gleicher tragender Fläche.

Gleiche Verdrängung pro Längeneinheit (exkl. Gewinde):

- Implantat mit 2 Knochenschrauben pro Wirbel mit Kerndurchmesser d = 5 mm:
Verdrängung pro Längeneinheit: $2$ x $d^2$ x Pi/4 x Länge / Länge = $39 \text{ mm}^2$ / mm
Tragende Fläche pro Längeneinheit: $2$ x d x Länge / Länge = $10 \text{ mm}^2$ / mm

- Implantat mit klingenförmigem Verankerungselement pro Wirbel mit Breite B = ? mm, Seele in der Mitte mit Durchmesser d = 5 mm und Klingendicke D = 1,2 mm: Verdrängung pro Längeneinheit: ((B-d) x H + $d^2$ x Pi/4) x Länge / Länge = $39 \text{ mm}^2$ / mm

Tragende Fläche pro Längeneinheit:
((39 mm$^2$ - d$^2$ x Pi/4) / H + d) x Länge / Länge = 21 mm$^2$ / mm

D.h. es resultiert eine Vergrösserung der tragenden Fläche um 110% bei gleicher Verdrängung.

2. Vorrichtungen, die mit Schrauben in den Wirbelkörpern verankert sind, bedingen mindestens 2 Knochenschrauben pro Wirbel oder eine Knochenschraube kombiniert mit einer agraffen-förmigen Unterlage, um die für die Fusion notwendige Rotationsstabilität zu erreichen. Ein klingenförmiges Verankerungselement ist in sich rotationsstabil im Knochen verankert und bietet dementsprechend die für eine erfolgreiche Fusion der überbrückten Wirbelkörper notwendige Rotationsstabilität und damit die Vorteile:

- Vorrichtungen, die kranial und kaudal über je ein klingenförmiges Verankerungselement mit der Wirbelsäule verbunden sind, können höher an der Wirbelsäule eingesetzt werden als die platzkonsumierenden, über Schrauben verankerten Implantate;

- Durch die Reduktion auf ein Verankerungselement pro Wirbel wird die Montage von auf klingenförmigen Verankerungselementen basierenden Implantaten vereinfacht und beschleunigt.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine Aufsicht auf eine Vorrichtung zur Fixation von Wirbelkörpern;

Fig. 2 eine Seitenansicht der in Fig. 1 dargestellten Vorrichtung;

Fig. 3 eine Seitenansicht einer weiteren Ausführungsform der Vorrichtung;

Fig. 4 eine perspektivische Ansicht einer Ausführungsform des Verankerungselementes;

Fig. 5a bis 5g Aufsichten auf verschiedene Ausführungsformen des Verankerungselementes;

Fig. 6a und 6b Seitenansichten auf verschiedene Ausführungsformen des Verankerungselementes;

Fig. 7a bis 7e Querschnitte von Verankerungssegmenten verschiedener Ausführungsformen des Verankerungselementes;

Fig. 8a und 8b perspektivische Ansichten von verwundenen Verankerungssegmenten in verschiedenen Ausführungsformen des Verankerungselementes;

Fig. 9a und 9b perspektivische Ansichten von aus mehreren Klingen bestehenden Verankerungssegmenten in verschiedenen Ausführungsformen des Verankerungselementes;

Fig. 10 eine perspektivische Ansicht einer Ausführungsform eines mit einer Transportschraube ausgestatteten spiralförmigen Verankerungselementes;

Fig. 11 eine perspektivische Ansicht einer weiteren Ausführungsform eines mit einer Transportschraube ausgestatteten Verankerungselementes mit 3 Klingen;

Fig. 12 eine perspektivische Ansicht einer weiteren Ausführungsform eines mit einer Transportvorrichtung ausgestatteten Verankerungselementes;

Fig. 13 eine perspektivische Ansicht von einem mit einer Transportvorrichtungen implantierbaren Verankerungselement;

Fig. 14a eine perspektivische Ansicht einer Ausführungsform des Verankerungselementes mit einer Oberflächenstruktur;

Fig. 14b eine perspektivische Ansicht einer anderen Ausführungsform des Verankerungselementes mit einer Oberflächenstruktur;

Fig. 14c eine perspektivische Ansicht einer Ausführungsform des Verankerungselementes mit quer verlaufenden Löchern;

Fig. 15 eine perspektivische Ansicht einer Ausführungsform eines mit einer Gewindehülse ausgestatteten Verankerungselementes;

Fig. 16a und 16b perspektivische Ansichten von Vorrichtungen zur Verankerung einer Transportvorrichtung zum Einbringen des Verankerungselementes;

Fig. 17a und 17b perspektivische Ansichten einer weiteren Vorrichtung zur Verankerung einer Transportvorrichtung zum Einbringen des Verankerungselementes;

Fig. 18 einen Querschnitt durch einen Wirbelkörper mit einem implantierten Verankerungselement; und

Fig. 19 eine Ansicht eines Auschnittes einer Wirbelsäule mit einer implantierten erfindungsgemässen Vorrichtung.

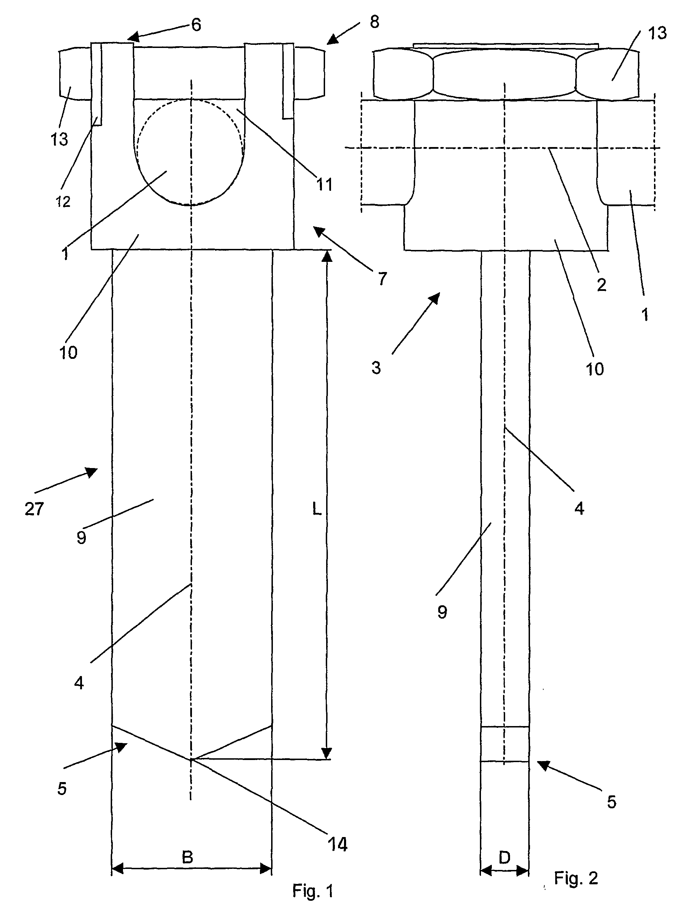

In Fig. 1 und 2 ist eine Vorrichtung dargestellt, welche ein Verankerungselement 3 mit Aufnahmemitteln 7 für einen Längsträger 1 am hinteren Ende 6 des Verankerungselementes 3 umfasst. Die Aufnahmemittel 7 bestehen aus einem im wesentlichen kreiszylindrischen, zur Längsachse 4 des Verankerungselementes 3 koaxialen Aufnahmekopf 10 mit einem gegen das hintere Ende 6 offenen Kanal 11 zur Aufnahme des Längsträgers 1. Der Längsträger 1 wird dabei so aufgenommen, dass seine Zentralachse 2 senkrecht zur Längsachse 4 verläuft. Das Verankerungselement 3 enthält in einem Bereich zwischen dem vorderen Ende 5 und dem Aufnahmekopf 10 ein Verankerungssegment 27, welches als im wesentlichen quaderförmige Klinge 9 ausgebildet ist. Die Klinge 9 weist senkrecht zur Längsachse 4 betrachtet einen rechteckigen Querschnitt mit einer Breite B und einer Dicke D auf. Die Breite B der Klinge 9 konvergiert am vorderen Ende 5 des Verankerungselementes 3 zu einer Spitze 14. Ferner sind als Feststellmittel 8 am hinteren Ende 6 des Verankerungselementes 3 ein zur Längsachse 4 konzentrisches Aussengewinde 12 auf dem Aufnahmekopf 10 und eine über dieses Aussengewinde 12 schraubbare Mutter 13 angeordnet, so dass beim Anziehen der Mutter 13 der Längsträger 1 im Kanal 11 axial festgeklemmt wird, wodurch das Verankerungselement 3 am Längsträger 1 blockiert wird.

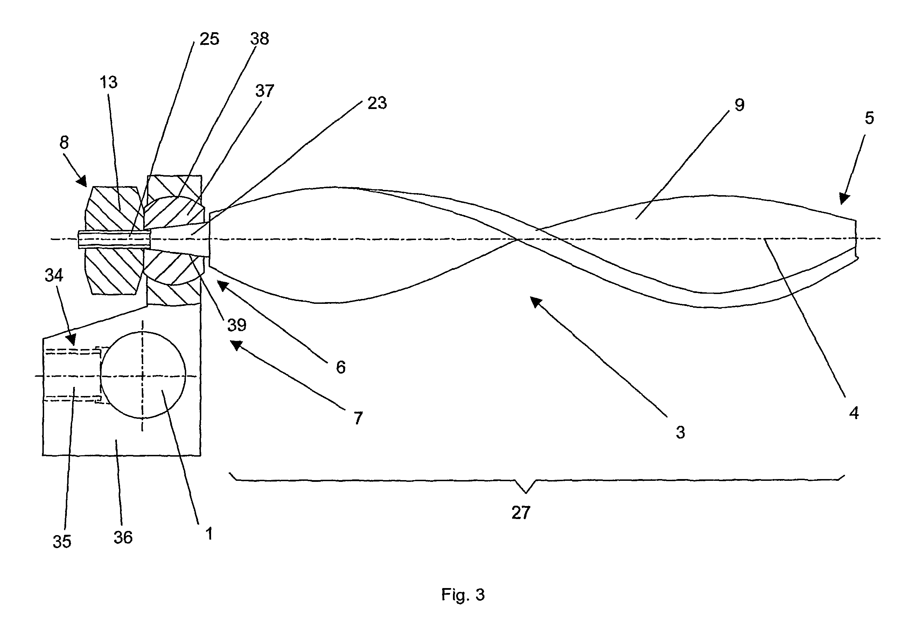

Die in Fig. 3 gezeigte Ausführungsform der Vorrichtung umfasst ein Verankerungselement 3, welches als Verankerungssegment 27 eine koaxial zur Längsachse 4 spiralförmige Klinge 9 aufweist. Am hinteren Ende 6 des Verankerungselementes 3 sind koaxial zur Längsachse 4 an das Verankerungssegment 27 angrenzend ein Konussegment 23 und ebenfalls koaxial daran anschliessend ein Gewindezapfen 25 angeordnet. Die Aufnahmemittel 7 bestehen im wesentlichen aus einem durchbohrten Verbindungselement 36, worin der Längsträger 1 koaxial zur Zentralachse 2 (Fig. 2) verschiebbar gelagert und mit einer Arretierschraube 35 feststellbar ist. Neben dem Längsträger 1 verlaufend ist im Verbindungselement 36 eine das Verbindungselement 36 durchdringende, hohlkugelförmige Kavität 38 angeordnet, worin ein kugelschichtförmiges, geschlitztes Spannelement 37 gelagert ist. Das Spannelement 37 ist mit einem Innenkonus 39 versehen, worin das Konussegment 23 am Verankerungselement 3 aufnehmbar ist. Durch die Verbindung zwischen Verankerungselement 3 und Verbindungselement 36 mittels des in der Kavität 38 um drei senkrecht aufeinander stehende Achsen rotierbaren Spannelementes 37 ist der Winkel zwischen der Zentralachse 2 des Längsträgers 1 und der Längsachse 4 des Verankerungselementes 3 variierbar. Mit einer Mutter 13, welche über den endständigen Gewindezapfen 25 schraubbar ist, wird das Konussegment 23 in den Innenkonus 39 des Spannelementes 37 gezogen, wodurch dieses radial gespreizt und in der Kavität 38 festgeklemmt wird, so dass das Verankerungselement 3 im Verbindungselement 36 blockiert wird. Eine solche Verbindung zwischen einem Längsträger 1 und einer Pedikelschraube als Verankerungselement 3 ist in der EP 0 599 847 SCHLÄPFER beschrieben. Anstelle des Konussegmentes 23 und des Gewindezapfens 25 kann am hinteren Ende 6 des Veankerungselementes 3 ein Kugelkopf angeordnet sein, welcher mittels einer Vorrichtung zur Verbindung eines Längsträgers 1 mit einem als Pedikelschraube ausgebildeten Verankerungselement 3 wie sie in der WO 98/52482 SCHLÄPFER offenbart ist, mit dem Längsträger 1 verbindbar ist.

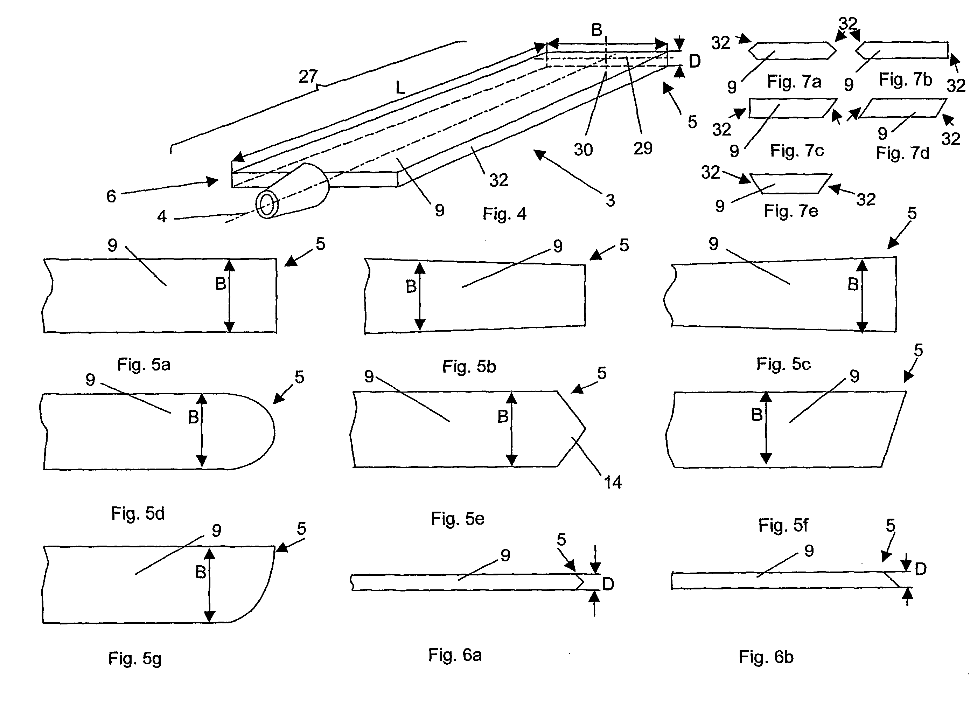

In Fig. 4 ist eine Ausführungsformen des an das hintere Ende 6 des Verankerungselementes 3.j auf einer Länge L angrenzenden Verankerungssegmentes 27 dargestellt. Das Verankerungssegment 27 besteht im wesentlichen aus einer flachen Klinge 9, welche eine Länge L, eine Breite B und eine Dicke D aufweist. Die Seitenflächen 32 der Klinge 9 werden durch die Länge L und die Dicke D eingeschlossen. Der senkrecht zur Längsachse 4 betrachtete Querschnitt der Klinge 9 umfasst eine erste Querachse 29, welche in der Ebene der Seitenflächen 32 verläuft und senkrecht auf der Längsachse 4 steht und eine zweite Querachse 30, welche senkrecht auf den Seitenflächen 32 und senkrecht auf der Längsachse 4 steht. Das Verhältnis von Breite B zu Dicke D beträgt im wesentlichen zwischen 1 und 14, vorzugsweise zwischen 3 und 6. Dank diesem Verhältnis von Breite B zu Dicke D kann das Implantat ohne grosse Zerstörung des Knochens in einen Wirbelkörper eingebracht werden. Die Klinge 9 wird dabei mit Berücksichtigung der Lasteinwirkung im Knochen ausgerichtet. Wird die Klinge 9 so in einem Wirbelkörper implantiert, dass die erste Querachse 29 parallel zur Längsachse der Wirbelsäule verläuft, hat die Klinge 9 eine hohe Festigkeit gegen Biegung, weist jedoch bezüglich Durchschneidung des Knochens eine ungünstige Position auf. Wird die Klinge 9 hingegen so in den Wirbelkörper implantiert, dass die zweite Querachse 30 parallel zur Längsachse der Wirbelsäule verläuft, ergibt sich bezüglich Durchschneidung des Knochens eine günstigere Position der Klinge 9, was allerdings mit einer geringeren Biegefestigkeit der Klinge 9 verbunden ist. Die Klinge 9 kann folgendermassen ausgestaltet sein:

a) quaderförmig mit einer Länge L, einer Dicke D und einer Breite B (Fig. 5a);
b) keilförmig mit einer gegen das vordere Ende 5 konvergierenden Breite B (Fig. 5b);
c) keilförmig mit einer gegen das vordere Ende 5 divergierenden Breite B (Fig. 5c);
d) in der Aufsicht betrachtet ist die Klinge 9 am vorderen Ende 5 konvex ausgebildet (Fig. 5d);

e) in der Aufsicht betrachtet verläuft die Klinge 9 am vorderen Ende 5 gleichseitig oder ungleichseitig in eine Spitze 14 (Fig. 5e);

f) in der Aufsicht betrachtet ist die Klinge 9 am vorderen Ende 5 einseitig abgeschrägt (Fig. 5f);

g) in der Aufsicht betrachtet ist die Klinge 9 am vorderen Ende 5 einseitig abgerundet (Fig. 5g);

h) in einem parallel zu den Seitenflächen 32 verlaufenden Längsschnitt ist die Klinge 9 am vorderen Ende 5 gleichseitig oder ungleichseitig zugespitzt (Fig. 6a);

i) in einem parallel zu den Seitenflächen 32 verlaufenden Längsschnitt ist die Klinge 9 am vorderen Ende 5 einseitig abgeschrägt (Fig. 6b);

j) in einem senkrecht zur Längsachse 4 stehenden Querschnitt verlaufen beide in den Seitenflächen 32 liegenden Seitenkanten der Klinge 9 in einen Spitz (Fig. 7a);

k) in einem senkrecht zur Längsachse 4 stehenden Querschnitt verläuft eine in einer Seitenfläche 32 liegende Seitenkante der Klinge 9 in einen Spitz (Fig. 7b);

l) in einem senkrecht zur Längsachse 4 stehenden Querschnitt ist eine in einer Seitenfläche 32 liegende Seitenkante der Klinge 9 abgeschrägt (Fig. 7c);

m) in einem senkrecht zur Längsachse 4 stehenden Querschnitt sind beide in den Seitenflächen 32 liegende Seitenkanten der Klinge 9 gleich abgeschrägt (Fig. 7d); und

n) in einem senkrecht zur Längsachse 4 stehenden Querschnitt sind beide in den Seitenflächen 32 liegende Seitenkanten der Klinge 9 gegengleich abgeschrägt (Fig. 7e).

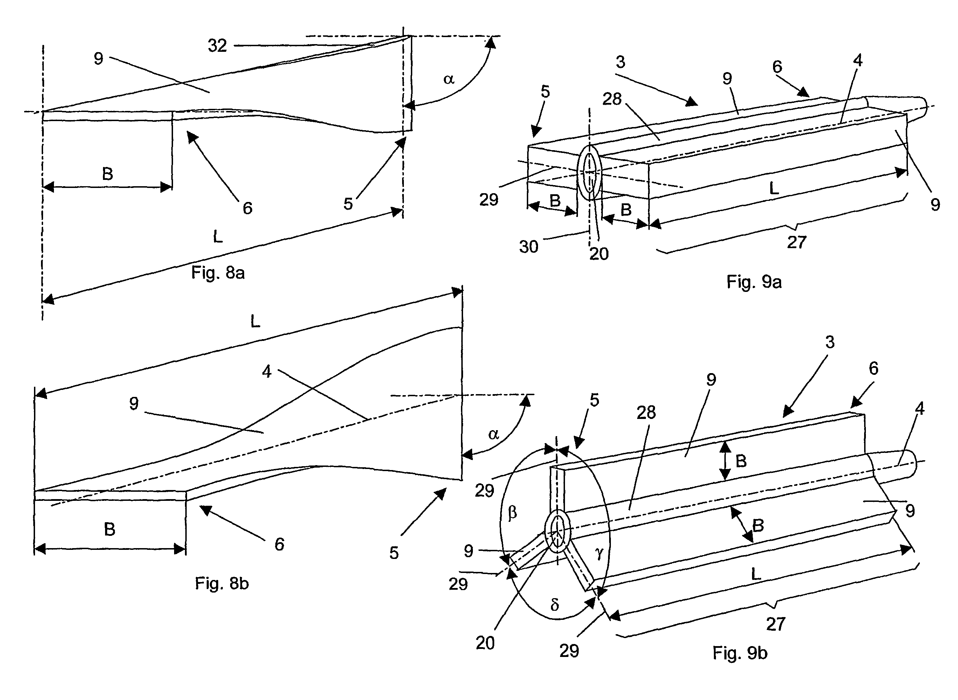

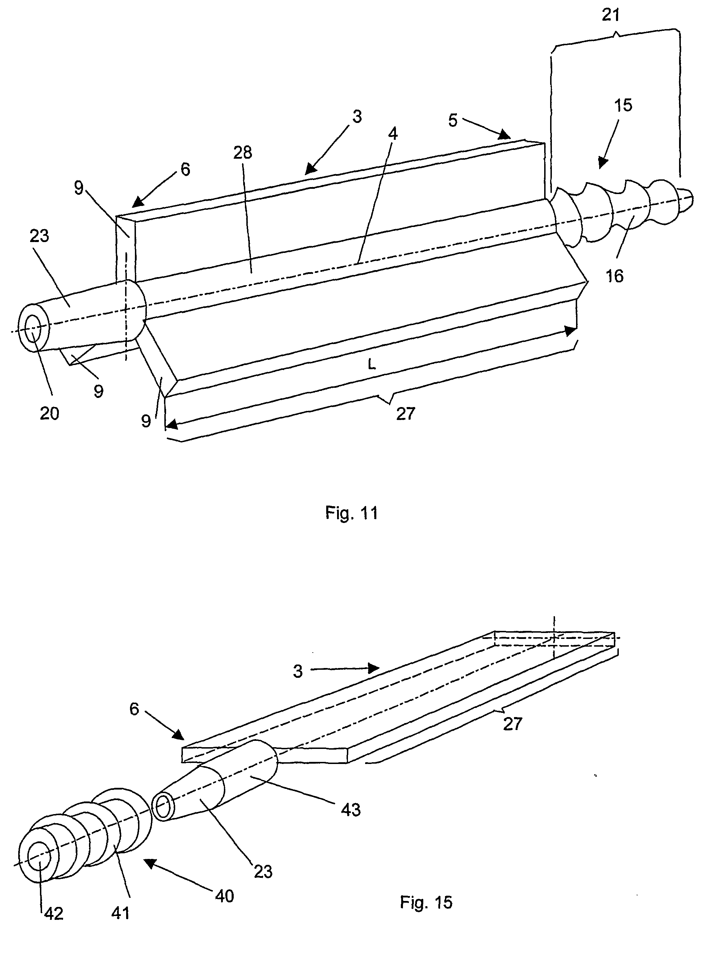

In Fig. 8a und 8b sind zwei Ausführungsformen einer verwundenen Klinge 9 dargestellt. Fig. 8a zeigt eine Klinge 9, welche auf der Länge L um eine die Länge L einschliessende, an eine der Seitenflächen 32 angrenzende Kante der Klinge 9 um einen Verdrehungswinkel $\alpha$ verwunden ist. Fig. 8b zeigt eine Klinge 9, welche auf der Länge L um die Längsachse 4 ebenfalls um einen Verdrehungswinkel $\alpha$ verwunden ist. Die Verwindung kann eine Linksverdrehung oder Rechtsverdrehung sein. Für die Verdrehungslänge $L_v$ und den Verdrehungswinkel $\alpha$ lässt sich die Steigung der Verdrehung wie folgt definieren:

$$\text{Steigung } S = L_v * 360° / \alpha \ [°]$$

Im Falle einer solchen spiralförmigen Ausbildung der Klinge 9 ist eine Steigung zwischen 60 mm bis 300 mm, vorzugsweise zwischen 100 mm und 240 mm vorteilhaft.

Fig. 9a zeigt eine weitere Ausführungsform des Verankerungselementes 3 mit einer Kombination von Klingen 9. Das Verankerungselement 3 umfasst ein Verankerungssegment 27 mit zwei Klingen 9, welche durch einen auf der gesamten Länge des Verankerungselementes 3 koaxial zur Längsachse 4 angeordneten Hohlkörper 28 verbunden werden. Die beiden Klingen 9 sind dabei so angeordnet, dass ihre ersten Querachsen 29 in einer Ebene liegen. Der Hohlkörper 28 ist mit einer Bohrung 20 konzentrisch zur Längsachse 4 durchbohrt.

Die in Fig. 9b dargestellte Ausführungsform des Verankerungselementes 3 ist eine weitere Kombination mehrerer Klingen 9, welche drei sternförmig angeordnete Klingen 9 umfasst. Die einen Seitenflächen 32 (Fig. 4) der drei Klingen 9 sind parallel zur Längsachse 4 mit einem koaxialen Hohlzylinder 28 verbunden, wobei die ersten Querachsen 29 der Klingen 9 bezüglich der Längsachse 4 die Zentriwinkel $\beta$, $\gamma$, $\delta$ einschliessen. In der hier dargestellten Ausführungsform sind die Zentriwinkel $\beta$, $\gamma$, $\delta$ gleich, d.h. $\beta = \gamma = \delta$, während in anderen Ausführungsformen die Klingen 9 mit unterschiedlichen Zentriwinkeln je nach Bedarf angeordnet sein können. Ein Verankerungselement 3, welches drei oder mehr Klingen 9 umfasst, kann auch spiralförmig ausgestaltet sein.

Bei Marknagelsystemen wird das klingenförmige Verankerungsimplantat eingehämmert. An der Wirbelsäule ist ein Einhämmern verpönt, da die Gefahr der Verletzung von lebenswichtigen neurologischen und vaskulären Strukturen besteht.

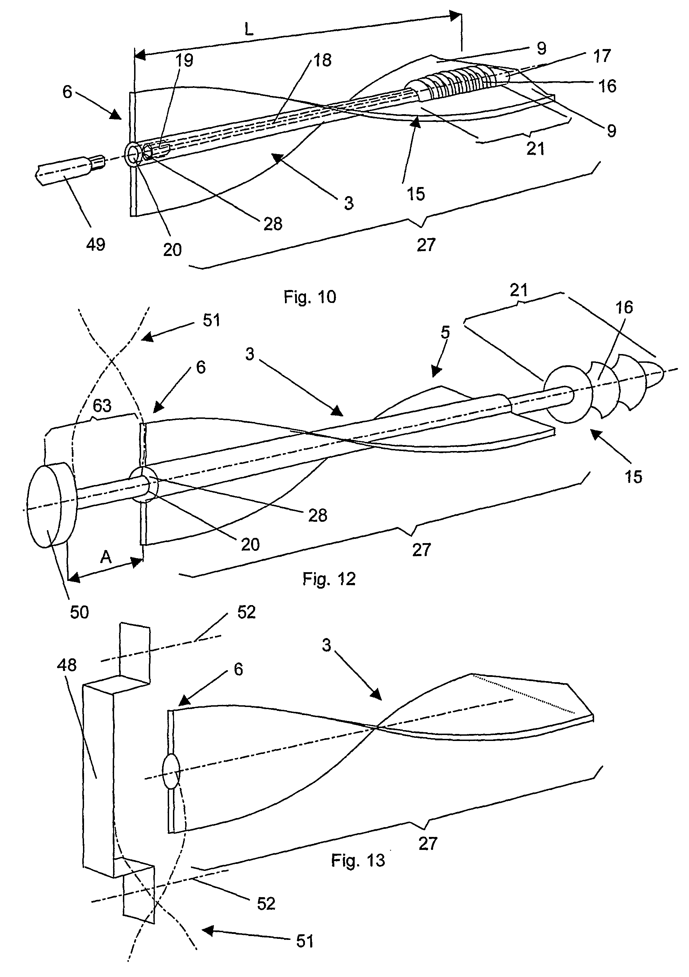

Möglichkeiten um das Verankerungselement 3 kontrolliert mittels einer Transportvorrichtung 15 einzubringen sind in den Fig. 10 bis 13 dargestellt. Die in Fig. 10 gezeigte Transportvorrichtung 15 umfasst eine Transportschraube 16 mit einer Schraubenspitze 17, einem Schraubenschaft 18, einem an die Schraubenspitze 17 angrenzenden Gewindesegment 21 und Antriebsmittel 19, wobei das Antriebsmittel 19 vom hinteren Ende 6 des Verankerungselementes 3 bedienbar ist. Die Transportschraube 16 ist relativ zum Verankerungselement 3 frei drehbar. Wenn die Transportschraube 16 mittels dem Schraubendreher 49 gedreht wird, schraubt sie sich durch den Knochen und zieht dabei das Verankerungselement 3 mit sich.

Das Gewindesegment 21 kann gemäss Fig. 10 und 11 in das Verankerungssegment 27 integriert sein, wobei die Gewindegänge radial über die Dicke D der Klinge 9 vorstehen, oder am vorderen Ende 5 des Verankerungselementes 3 über das Verankerungssegment 27 endständig hinausragen. Ferner kann das Gewindesegment 21 sich nur über einen Teil der Länge L oder über die gesamte Länge L erstrecken. Die Vorteile dieser Transportvorrichtung 15 liegen darin, dass die Transportschraube 16 das Verankerungssegment 27 direkt in den Knochen hineinzieht. Nachteilig hingegen ist, dass in der Mitte des Wirbelkörpers der Knochen sehr porös ist, so dass die Transportschraube 16 ausreissen und die Transportvorrichtung versagen könnte.

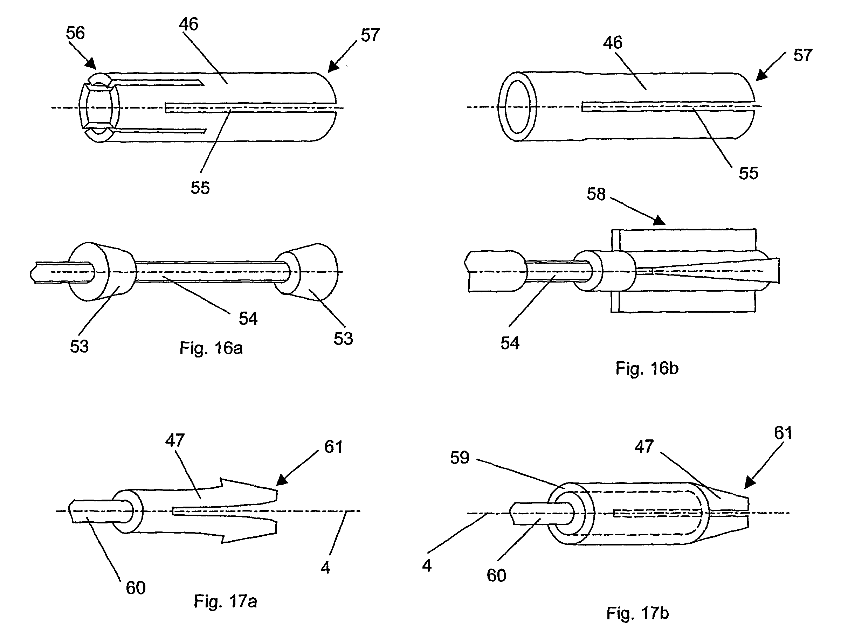

In Fig. 12 umfasst die Transportvorrichtung 15 eine Transportschraube 16, welche in der Bohrung 20 im Hohlkörper 28 gelagert ist, über das vordere Ende 5 des Verankerungselementes 3 hinausragt und in der Gegenkortikalis verankerbar ist. Anstelle einer Verankerung in der Gegenkortikalis mittels eines Gewindes, wie im Fall der Transportschraube 16, können auch andere Elemente wie Dübel 46 (Fig. 16a und 16b) oder Haken 47 (Fig. 17a und 17b) etc. mit Verlängerung eingesetzt werden. Am hinteren Ende 6 des Verankerungselementes 3 weist die Transportvorrichtung 15 eine mit der Transportschraube 16 verbundene Verlängerung 63 auf, die über das Längensegment A koaxial über das Verankerungselement 3 hinausragt und endständig ein Widerlager 50 aufweist, so dass eine Spreizvorrichtung 51 zwischen das Widerlager 50 und das hintere Ende 6 des Verankerungselementes 3 eingefügt und das Verankerungssegment 27 durch Spreizen der Spreizvorrichtung 51 in den Knochen eingepresst werden kann. Die Vorteile dieser Ausführungsform der Transportvorrichtung 15 liegen darin, dass die Transportschraube 16 zum vorneherein im best möglichen Knochen verankert ist. Nachteilig hingegen ist das durch die Grössenbegrenzung bedingte aufwendig Instrumentarium.

Die in Fig. 13 symbolisch gezeigte U-förmige Hilfsvorrichtung 48 wird z.B. durch Knochenschrauben 52 in der Kortikalis als Widerlager verankert, um das Verankerungssegment 27 am hinteren Ende 6 des Verankerungselementes 3 mittels einer Spreizvorrichtung 51 in den Knochen zu drücken. Nachteilig wirkt hier die für die Verankerung der Hilfsvorrichtung 48 notwendige zusätzliche Durchlöcherung der Wirbelkörper.

Im Vergleich zu Knochenschrauben wie auch zu Hohlschrauben hat die Klinge 9 den Nachteil, dass die Verankerungsfestigkeit in der Längsachse 4 relativ gering ist. Die in den Fig. 10 bis 12 gezeigten Transportvorrichtungen 15 erhöhen die Verankerungsfestigkeit.

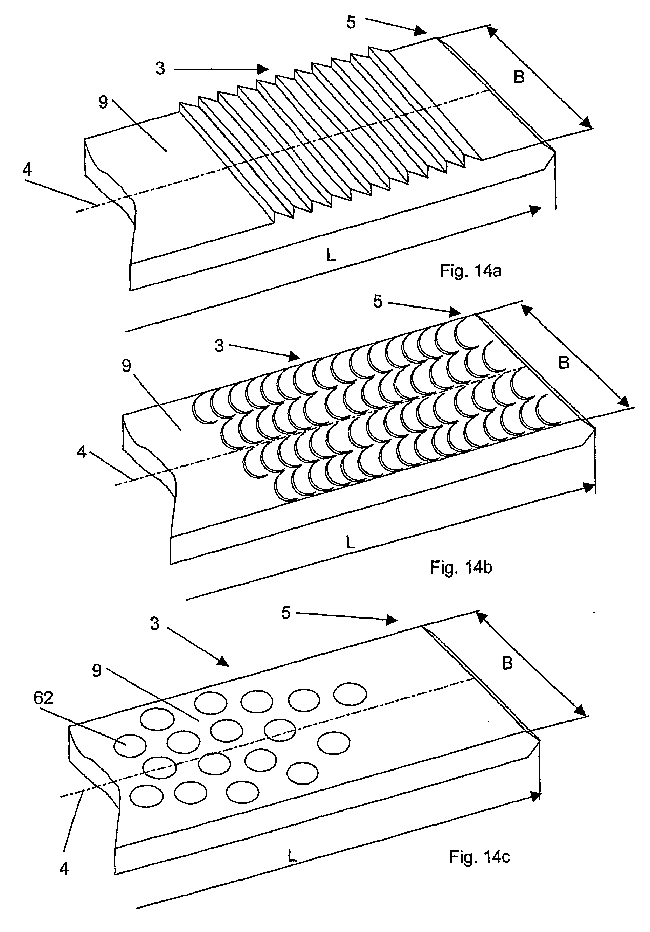

Fig. 14a bis 14c zeigen Möglichkeiten, wie durch die Gestaltung der Oberfläche der Klinge 9 die Verankerungsfestigkeit in der Längsachse 4 erhöht werden kann:

a) sägezahnartige Ausbildung der durch die Länge L und die Breite B eingeschlossenen Oberfläche der Klinge 9 (Fig. 14a), wobei die steilen Seiten der Sägezähne von dem vorderen Ende 5 des Verankerungselementes 3 weggerichtet sind; oder

b) schuppenartige Ausbildung der durch die Länge L und die Breite B eingeschlossenen Oberfläche der Klinge 9 (Fig. 14b), wobei auch hier die steilen Seiten der Schuppen von dem vorderen Ende 5 des Verankerungselementes 3 weggerichtet sind.

Dabei kann die sägenzahnartige oder schuppenartige Ausbildung nur auf einer der durch die Länge L und die Breite B eingeschlossenen Oberflächen oder auf beiden diesen Oberflächen angebracht sein, nur einen Teil des Länge L einnehmen oder sich über die gesamte Länge L erstrecken.

c) Anstelle einer mechanischen Verriegelung ist auch eine biologische Verriegelung einsetzbar. Wie in Fig. 14c gezeigt wird in diesem Fall die Klinge 9 senkrecht zu den durch die Länge L und die Breite B eingeschlossenen Oberflächen mit mehreren Löchern 62 durchbohrt, so dass der Knochen durch die Löcher 62 wachsen kann.

In der Ausführungsform gemäss Fig. 15 ist am hinteren Ende 6 des Verankerungselementes 3 eine Gewindehülse 40 mit einem Aussengewinde 41 angeordnet. Die Gewindehülse 40 umfasst eine koaxiale Bohrung 42, welche über einen zwischen dem Verankerungssegment 27 und dem endständigen Konussegment 23 angeordneten Zylinderzapfen 43 schiebbar ist. Wie die Transportschraube 16 (Fig. 10 bis 12) dient diese Gewindehülse 40 ebenfalls zur Erhöhung der Verankerungsfestigkeit des Verankerungselementes 3 im Knochen. Diese Ausführungsform kann in Kombination mit den in den Fig. 10 bis 14 gezeigten Versionen angewendet werden.

In den Fig. 16a und 16b sind Vorrichtungen zur Verankerung der Transportvorrichtung 16 (Fig. 12) in der Gegenkortikalis dargestellt, welche anstelle einer Transportschraube 15 (Fig. 12) einsetzbar sind. Dabei handelt es sich um hohlzylindrische Dübel 46, welche von ihrem ersten Ende 56 her und von ihrem zweiten Ende 57 her durch koaxiale Schlitze 55 radial elastisch ausgestaltet sind (Fig. 16a). In der Ausführungsform nach Fig. 16a erfolgt die radiale Spreizung der Dübel 46 mittels zwei koaxial auf einer ebenfalls koaxialen Gewindestange 54 angeordneten Spreizkonusse 53, welche durch das Aussengewinde auf der Gewindestange und entsprechende Innengewinde in den Spreizkonussen 53 gegeneinander verschiebbar sind. Wie in Fig. 16b gezeigt kann anstelle der Spreizkonusse 53 auch ein Keilelement 58 zur radialen Spreizung des Dübels 46 eingesetzt werden. Hier dargestellt ist ein Dübel 46, welcher nur vom zweiten Ende 57 her koaxial eindringende Schlitze 55 aufweist, so dass die Spreizung des Dübels 46 durch koaxiales Einziehen des Keilelementes 58 mittels der Gewindestange 54 erfolgt. Das Keilelement 58 kann anstatt 4-teilig, wie in Fig. 16b dargestellt auch 1-, 2- oder 3-teilig sein.

Eine weitere Vorrichtung zur Verankerung der Transportvorrichtung 16 (Fig. 12) in der Gegenkortikalis ist in den Fig. 17a und 17b dargestellt. Hier handelt es sich um einen bezüglich der Längsachse 4 des Verankerungselementes 3 (Fig. 1) radial sich spreizenden Haken 47, welcher sich gegen seine Spitze 61 verjüngt und vor sowie während der Montage durch eine koaxial angeordnete Hülse 59 radial zusammengepresst wird. Eingeführt wird der Haken 47 mittels einer koaxialen Stange 60. Nach dem Einführen des Hakens 47 wird die Hülse 59 vom Haken 47 über sein der Spitze 61 entgegengesetztes Ende weggezogen, worauf sich der Haken 47 elastisch radial spreizt und damit in der Gegenkortikalis verankert wird.

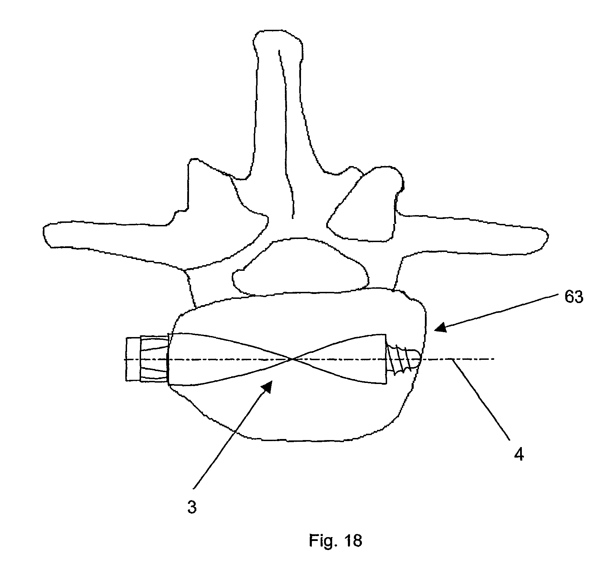

Fig. 18 zeigt einen Querschnitt durch einen Wirbelkörper 63 mit einem eingesetzten Verankerungselement 3, welches den Wirbelkörper 63 mit einer quer zur Wirbelsäulenlängsachse verlaufenden Längsachse 4 durchdringt.

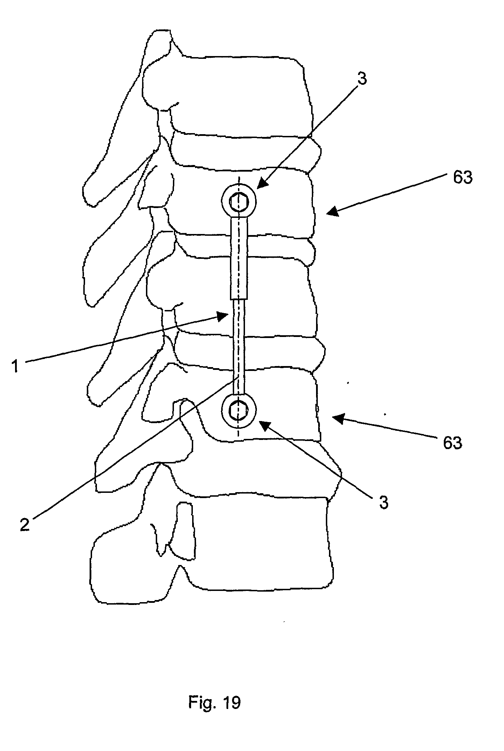

Fig. 19 zeigt eine Seitenansicht mehrerer Wirbelkörper 63 mit einer implantierten erfindungsgemässen Vorrichtung, welche in dieser Ausführungsform einen teleskopierbaren Längsträger 1 mit einer parallel zur Wirbelsäulenlängsachse verlaufenden Zentralachse 2 und zwei Verankerungselemente 3 umfasst.

## Patentansprüche

1. Vorrichtung zur Fixation von Knochen, insbesondere von Wirbelkörpern relativ zueinander umfassend

   A) einen longitudinalen Längsträger (1) mit einer Zentralachse (2); und
   B) n Verankerungselemente (3.i) ($2 \leq i \leq n$) mit den Längsachsen (4), je einem vorderen Ende (5) und je einem hinteren Ende (6), wobei
   C) die Längsachsen (4) der Verankerungselemente (3.i) in einem Winkel zwischen 65° und 115° zur Zentralachse (2) des Längsträgers (1) stehen; und
   D) mindestens eines der Verankerungselemente (3.j) ($1 \leq j \leq n$) klingenförmig ausgebildet ist, und
   E) mindestens das Verankerungselement (3.j) am hinteren Ende (6) Aufnahmemittel (7) mit einer Aufnahme (11) für den Längsträger (1) mit Feststellmitteln (8;34) zur lösbaren Arretierung der Verbindung zwischen Längsträger (1) und Verankerungselement (3.j) umfasst, und
   F) die arretierte Verbindung keine Relativbewegungen zwischen Längsträger (1) und Verankerungselement (3.j) zulässt sowie Kräfte und Momente in allen drei Achsrichtungen eines dreidimensionalen Koordinatensystems aufnimmt,
   **dadurch gekennzeichnet, dass**
   G) mindestens das Verankerungselement (3.j) eine Transportvorrichtung (15) zum Einbringen des Verankerungselementes (3.j) parallel zur Längsachse (4) in einen Knochen umfasst.

2. Vorrichtung nach Anspruch 1, **dadurch gekennzeichnet, dass** das mindestens eine Verankerungselement (3.j) ($1 \leq j \leq n$) an das hintere Ende (6) des Verankerungselementes (3.j) angrenzend klingenförmig ausgebildet ist.

3. Vorrichtung nach Anspruch 1 oder 2, **dadurch gekennzeichnet, dass** die Feststellmittel (8) vom hinteren Ende (6) des mindestens einen Verankerungselementes (3.j) bedienbar sind.

4. Vorrichtung nach einem der Ansprüche 1 bis 3, **dadurch gekennzeichnet, dass** die Aufnahme (11) seitlich offen ist, so dass der Längsträger (1) quer zur Längsachse (4) in die Aufnahme (11) einführbar ist.

5. Vorrichtung nach einem der Ansprüche 1 bis 3, **dadurch gekennzeichnet, dass** die Aufnahme (7) vom hinteren Ende (6) her offen ist, so dass der Längsträger (1) parallel zur Längsachse (4) in die Aufnahme (11) einführbar ist.

6. Vorrichtung nach einem der Ansprüche 1 bis 5, **dadurch gekennzeichnet, dass** die Transportvorrichtung (15) als Transportschraube (16).ausgeführt ist.

7. Vorrichtung nach Anspruch 6, **dadurch gekennzeichnet, dass** die Transportschraube (16) eine Schraubenspitze (17), einen Schraubenschaft (18), ein an die Schraubenspitze (17) angrenzendes Gewindesegment (21) und Antriebsmittel (19) umfasst.

8. Vorrichtung nach Anspruch 7, **dadurch gekennzeichnet, dass** die Antriebsmittel (19) vom hinteren Ende (6) des Verankerungselementes (3.j) aus bedienbar sind.

9. Vorrichtung nach Anspruch 8, **dadurch gekennzeichnet, dass** mindestens ein Verankerungselement (3.j) koaxial eine durchgehende Bohrung (20) aufweist und die Transportschraube (16) in dieser Bohrung (20) aufnehmbar ist, so dass die Schraubenspitze (17) und das Gewindesegment (21) axial über das vordere Ende (5) des Verankerungselementes (3.j) vorstehen.

10. Vorrichtung nach Anspruch 8, **dadurch gekennzeichnet, dass** mindestens eine Verankerungselement (3.j) koaxial eine durchgehende Bohrung (20) aufweist und die Transportschraube (16) in dieser Bohrung (20) aufnehmbar ist, wobei das Gewindesegment (21) im Verankerungselement (3.j) integriert ist und radial auf einem Teil seines Umfanges senkrecht zur Längsachse (4) über das Verankerungselement (3.i) vorsteht.

11. Vorrichtung nach einem der Ansprüche 1 bis 10, **dadurch gekennzeichnet, dass** das hintere Ende (6) von mindestens einem der Verankerungselemente (3.j) ein koaxiales Konussegment (23) aufweist.

12. Vorrichtung nach Anspruch 11, **dadurch gekennzeichnet, dass** das Konussegment (23) eine konzentrische, axial endständig offene Gewindebohrung (24) aufweist.

**13.** Vorrichtung nach Anspruch 11, **dadurch gekennzeichnet, dass** an das Konussegment (23) axial endständig ein konzentrischer Zapfen (25) mit einem Aussengewinde anschliesst.

**14.** Vorrichtung nach einem der Ansprüche 1 bis 10, **dadurch gekennzeichnet, dass** die Aufnahmemittel (7) einen an das hintere Ende (6) des Verankerungselementes (3.j) endständig angrenzenden koaxialen Kugelkopf (26) umfassen.

**15.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche einen im wesentlichen rechteckigen Querschnitt mit einer Dicke (D) und einer Breite (B) aufweist und die Klinge (9) eine erste Querachse (22) parallel zu den langen Seiten des Querschnittes umfasst.

**16.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer in der Aufsicht betrachtet gegen das vordere Ende (5) konvergierenden Klinge (9) umfasst.

**17.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer in der Aufsicht betrachtet gegen das vordere Ende (5) divergierenden Klinge (9) umfasst.

**18.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche in der Aufsicht betrachtet am vorderen Ende (9) zu einer Spitze (14) konvergiert.

**19.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche in der Aufsicht betrachtet am vorderen Ende (5) einseitig abgeschrägt ist.

**20.** Vorrichtung nach einem der Ansprüche 1 bis 14, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche in der Aufsicht betrachtet am vorderen Ende (5) konvex ist.

**21.** Vorrichtung nach einem der Ansprüche 1 bis 20, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche im Längsschnitt betrachtet am vorderen Ende (5) zu einer Spitze konvergiert.

**22.** Vorrichtung nach einem der Ansprüche 1 bis 20, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche im Längsschnitt betrachtet am vorderen Ende (5) einseitig angefast ist.

**23.** Vorrichtung nach einem der Ansprüche 1 bis 22, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche im Querschnitt betrachtet an mindestens einer Seitenfläche (32) zugespitzt ist.

**24.** Vorrichtung nach einem der Ansprüche 1 bis 22, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche im Querschnitt betrachtet an mindestens einer Seitenfläche (32) einseitig angefast ist.

**25.** Vorrichtung nach einem der Ansprüche 1 bis 24, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche auf der Länge (L) um eine an eine Seitenfläche (32) angrenzende Kante verwunden ist.

**26.** Vorrichtung nach einem der Ansprüche 1 bis 24, **dadurch gekennzeichnet, dass** das Verankerungselement (3.j) an das hintere Ende (6) angrenzend auf einer Länge (L) ein Verankerungssegment (27) mit mindestens einer Klinge (9) umfasst, welche auf der Länge (L) um die Längsachse (4) verwunden ist.

**27.** Vorrichtung nach einem der Ansprüche 1 bis 26, **dadurch gekennzeichnet, dass** das Verankerungselement (3.j) an das hintere Ende (6) angrenzend auf einer Länge (L) ein Verankerungssegment (27) mit zwei Klingen (9) umfasst.

**28.** Vorrichtung nach Anspruch 27, **dadurch gekenn-**

**zeichnet, dass** die Klingen (9) im Querschnitt betrachtet in einer Ebene liegen.

29. Vorrichtung nach Anspruch 27 oder 28, **dadurch gekennzeichnet, dass** die Klingen (9) parallel zur Längsachse (4) durch einen Stab getrennt sind.

30. Vorrichtung nach Anspruch 29, **dadurch gekennzeichnet, dass** der Stab ein parallel zur Längsachse (4) durchbohrter Hohlkörper 28 ist.

31. Vorrichtung nach einem der Ansprüche 1 bis 26, **dadurch gekennzeichnet, dass** das Verankerungselement (3.j) an das hintere Ende (6) angrenzend auf einer Länge (L) ein Verankerungssegment (27) mit drei oder mehr Klingen (9) umfasst und das Verankerungssegment (27) einen sternförmigen Querschnitt aufweist.

32. Vorrichtung nach einem der Ansprüche 1 bis 26, **dadurch gekennzeichnet, dass** das Verankerungselement (3.j) an das hintere Ende (6) angrenzend auf einer Länge (L) ein Verankerungssegment (27) mit drei oder mehr Klingen (9) umfasst, wobei die Klingen (9) im Querschnitt des Verankerungssegmentes (27) betrachtet mit ungleichen Zentriwinkeln angeordnet sind.

33. Vorrichtung nach einem der Ansprüche 1 bis 32, **dadurch gekennzeichnet, dass** das Verankerungselement (3.j) einen koaxialen Hohlkörper (28) mit einer ebenfalls koaxialen, das Verankerungselement (3.j) vom vorderen Ende (5) bis zum hinteren Ende (6) durchdringenden Bohrung (20) umfasst.

34. Vorrichtung nach einem der Ansprüche 1 bis 33, **dadurch gekennzeichnet, dass** das Verankerungselement (3.j) an das hintere Ende (6) angrenzend auf einer Länge (L) ein Verankerungssegment (27) mit mindestens einer Klingen (9) umfasst und die mindestens eine Klinge (9) eine sägezahnartige Oberflächenstruktur aufweist, wobei die steilen Seiten der Sägezähne gegen das hintere Ende (6) des Verankerungselementes (3.j) gerichtet sind.

35. Vorrichtung nach Anspruch 34, **dadurch gekennzeichnet, dass** die Oberflächenstruktur einseitig angebracht ist.

36. Vorrichtung nach Anspruch 34, **dadurch gekennzeichnet, dass** die Oberflächenstruktur beidseitig angebracht ist.

37. Vorrichtung nach einem der Ansprüche 1 bis 33, **dadurch gekennzeichnet, dass** das Verankerungselement (3.j) an das hintere Ende (6) angrenzend auf einer Länge (L) ein Verankerungssegment (27) mit mindestens einer Klingen (9) umfasst und die mindestens eine Klinge (9) eine schuppenartige Oberflächenstruktur aufweist, wobei die steilen Seiten der Schuppen gegen das hintere Ende (6) des Verankerungselementes (3.j) gerichtet sind.

38. Vorrichtung nach Anspruch 37, **dadurch gekennzeichnet, dass** die Oberflächenstruktur einseitig angebracht ist.

39. Vorrichtung nach Anspruch 37, **dadurch gekennzeichnet, dass** die Oberflächenstruktur beidseitig angebracht ist.

40. Vorrichtung nach einem der Ansprüche 15 bis 30 oder 33 bis 39, **dadurch gekennzeichnet, dass** das Verankerungssegment (27) in Querschnitt betrachtet zwei in einer Ebene liegende Klingen (9) umfasst, welche koaxial zur Längsachse (4) durch einen Hohlkörper (28) getrennt sind, wobei die Klingen (9) eine Dicke (D) zwischen 0,8 mm und 2 mm, eine Breite (B) zwischen 2,5 mm und 4,5 mm aufweisen und der Hohlzylinder (28) einen Durchmesser (d) zwischen 3 mm und 7 mm aufweist.

41. Vorrichtung nach einem der Ansprüche 15 bis 40, **dadurch gekennzeichnet, dass** das Verhältnis von Breite (B) zu Dicke (D) im wesentlichen zwischen 1 und 14 beträgt.

42. Vorrichtung nach Anspruch 41, **dadurch gekennzeichnet, dass** das Verhältnis von Breite (B) zu Dicke (D) im wesentlichen zwischen 3 und 6 beträgt.

43. Vorrichtung nach einem der Ansprüche 15 bis 42, **dadurch gekennzeichnet, dass** mindestens eine Klinge (9) spiralförmig ausgebildet ist.

44. Vorrichtung nach Anspruch 43, **dadurch gekennzeichnet, dass** die mindestens eine Klinge (9) eine Steigung S zwischen 60 mm bis 300 mm aufweist.

45. Vorrichtung nach Anspruch 44, **dadurch gekennzeichnet, dass** die mindestens eine Klinge (9) eine Steigung S zwischen 100 mm bis 240 mm aufweist.

46. Vorrichtung nach einem der Ansprüche 43 bis 45, **dadurch gekennzeichnet, dass** der Verdrehwinkel $\alpha$ über der Länge (L) der Klingen (9) zwischen 0° und 360° beträgt.

47. Vorrichtung nach Anspruch 46, **dadurch gekennzeichnet, dass** der Verdrehwinkel $\alpha$ über der Länge (L) der Klingen (9) zwischen 0° und 180° beträgt.

48. Vorrichtung nach Anspruch 47, **dadurch gekennzeichnet, dass** der Verdrehwinkel $\alpha$ über der Länge (L) der Klingen (9) zwischen 0° und 45° beträgt.

**49.** Vorrichtung nach Anspruch 47, **dadurch gekennzeichnet, dass** der Verdrehwinkel $\alpha$ über der Länge (L) der Klingen (9) zwischen 45° und 90° beträgt.

**50.** Vorrichtung nach einem der Ansprüche 15 bis 49, **dadurch gekennzeichnet, dass** mindestens eine Klinge (9) spiralförmig verdreht ist und die Verdrehung im Uhrzeigersinn verläuft.

**51.** Vorrichtung nach einem der Ansprüche 15 bis 49, **dadurch gekennzeichnet, dass** mindestens eine Klinge (9) spiralförmig verdreht ist und die Verdrehung im Gegenuhrzeigersinn verläuft.

**Claims**

**1.** A device for fixation of bones, especially vertebral bodies, relative to one another, comprising:

A) a longitudinal support (1) with a central axis (2); and
B) n anchoring elements (3.i) ($2 \leq i \leq n$) with the longitudinal axes (4), one front end each (5) and one back end each (6), whereby
C) the longitudinal axes (4) of the anchoring elements (3.i) are arranged at an angle of between 65° and 115° relative to the central axis (2) of the longitudinal support (1); and
D) at least one of the anchoring elements (3.j) ($1 \leq j \leq n$) is designed in the shape of a blade, and
E) at the back end (6) at least the anchoring element (3.j) comprises receiving means (7) with receiving means (11) for acceptance of the longitudinal support (1) with stopping means (8; 34) for reversibly securing the connection between the longitudinal support (1) and anchoring element (3.j), and
F) the secured connection does not permit any relative movement between the longitudinal support (1) and anchoring element (3.j), as well as taking up forces and moments in all three axial directions of a three-dimensional coordinate system
**characterised in that**
G) at least the anchoring element (3.j) comprises a transport device (15) for inserting the anchoring element (3.j) into a bone parallel to the longitudinal axis (4).

**2.** The device according to claim 1, wherein at least the one anchoring element (3.j) ($1 \leq j \leq n$) is designed in the shape of a blade abutting the back end (6) of the anchoring element (3.j).

**3.** The device according to claim 1 or 2, wherein the stopping means (8) can be operated from the back end (6) of at least the one anchoring element (3.j).

**4.** The device according to one of the claims 1 to 3, wherein the receiving means (11) is open at the side so that the longitudinal support (1) can be inserted into the receiving means (11) transverse to the longitudinal axis (4).

**5.** The device according to one of the claims 1 to 3, wherein the receiving means (11) is open from the back end (6) so that the longitudinal support (1) can be inserted into the receiving means (11) parallel to the longitudinal axis (4).

**6.** The device according to one of the claims 1 to 5, wherein the transport device (15) is designed as a transport screw (16).

**7.** The device according to claim 6, wherein the transport screw (16) comprises a screw tip (17), a screw shaft (18), a threaded segment (21) that abuts the screw tip (17), and drive means (19).

**8.** The device according to claim 7, wherein the drive means (19) can be operated from the back end (6) of the anchoring element (3.j).

**9.** The device according to claim 8, wherein at least one anchoring element (3.j) has a through hole (20) coaxially and the transport screw (16) can be accommodated in this hole (20) in such a way that the screw tip (17) and threaded segment (21) protrude axially over the front end (5) of the anchoring element (3.j).

**10.** The device according to claim 8, wherein at least one anchoring element (3.j) has a through hole (20) coaxially and the transport screw (16) can be accommodated in this hole (20), whereby the threaded segment (21) is integrated into the anchoring element (3.j) and over a part of its circumference protrudes radially over the anchoring element (3.j) vertical to the longitudinal axis (4).

**11.** The device according to one of the claims 1 to 10, wherein the back end (6) of at least one of the anchoring elements (3.j) has a coaxial cone segment (23).

**12.** The device according to claim 11, wherein the cone segment (23) has a concentric threaded hole (24) that is open at the end axially.

**13.** The device according to claim 11, wherein a concentric pin (25) with external threading axially abuts the cone segment (23) at the end.

**14.** The device according to one of the claims 1 to 10, wherein the receiving means (7) comprise a coaxial ball head (26) that abuts the back end (6) of the anchoring element (3.j) at the end.

15. The device according to one of the claims 1 to 14, wherein the anchoring element (3.j) has an anchoring segment (27) that has at least one blade (9) and abuts the back end (6) over a length (L), whereby said blade has an essentially rectangular cross-section with a thickness (D) and a width (B) and the blade (9) has a first transverse axis (22) parallel to the long sides of the cross-section.

16. The device according to one of the claims 1 to 14, wherein the anchoring element (3.j) has an anchoring segment (27) that has at least one blade (9) and abuts the back end (6) over a length (L), whereby said blade, when viewed from above, converges toward the front end (5).

17. The device according to one of the claims 1 to 14, wherein the anchoring element (3.j) has an anchoring segment (27) that has at least one blade (9) and abuts the back end (6) over a length (L), whereby said blade, when viewed from above, diverges toward the front end (5).

18. The device according to one of the claims 1 to 14, wherein the anchoring element (3.j) has an anchoring segment (27) that has at least one blade (9) and abuts the back end (6) over a length (L), whereby said blade, when viewed from above, converges to a point (14) at the front end (5).

19. The device according to one of the claims 1 to 14, wherein the anchoring element (3.j) has an anchoring segment (27) that has at least one blade (9) and abuts the back end (6) over a length (L), whereby said blade, when viewed from above, slopes down downward on one side at the front end (5).

20. The device according to of the claims 1 to 14, wherein the anchoring element (3.j) has an anchoring segment (27) that has at least one blade (9) and abuts the back end (6) over a length (L), whereby said blade, when viewed from above, is convex at the front end (5).

21. The device according to one of the claims 1 to 20, wherein the anchoring element (3.j) has an anchoring segment (27) that has at least one blade (9) and abuts the back end (6) over a length (L), whereby said blade, when viewed in longitudinal section, converges to a tip at the front end (5).

22. The device according to one of the claims 1 to 20, wherein the anchoring element (3.j) has an anchoring segment (27) that has at least one blade (9) and abuts the back end (6) over a length (L), whereby said blade, when viewed in longitudinal section, is bevelled at the front end (5) on one side.

23. The device according to one of the claims 1 to 22, wherein the anchoring element (3.j) has an anchoring segment (27) that has at least one blade (9) and abuts the back end (6) over a length (L), whereby said blade, when viewed in cross-section, narrows to a point on at least one lateral surface (32).

24. The device according to one of the claims 1 to 22, wherein the anchoring element (3.j) has an anchoring segment (27) that has at least one blade (9) and abuts the back end (6) over a length (L), whereby said blade, when viewed in cross-section, is bevelled on one side on at least one lateral surface (32).

25. The device according to one of the claims 1 to 24, wherein the anchoring element (3.j) has an anchoring segment (27) that has at least one blade (9) and abuts the back end (6) over a length (L), whereby said blade is twisted over length (L) around an edge that abuts one lateral surface (32).

26. The device according to one of the claims 1 to 24, wherein the anchoring element (3.j) has an anchoring segment (27) that has at least one blade (9) and abuts the back end (6) over a length (L), whereby said blade is twisted over length (L) around the longitudinal axis (4).

27. The device according to one of the claims 1 to 26, wherein the anchoring element (3.j) has an anchoring segment (27) that has two blades (9) and abuts the back end (6) over a length (L).

28. The device according to claim 27, wherein, when viewed in cross-section, the blades (9) lie in one plane.

29. The device according to claims 27 or 28, wherein, parallel to the longitudinal axis (4), the blades (9) are separated by a rod.

30. The device according to claim 29, wherein the rod is a hollow (28) that is drilled parallel to the longitudinal axis (4).

31. The device according to one of the claims 1 to 26, wherein the anchoring element (3.j) has an anchoring segment (27) that has three or more blades (9) and abuts the back end (6) over a length (L) and the anchoring segment (27) has a star-shaped cross-section.

32. The device according to one of the claims 1 to 26, wherein the anchoring element (3.j) has an anchoring segment (27) has three or more blades (9) and abuts the back end (6) over a length (L), whereby the blades (9), viewed in the cross-section of the anchoring segment (27), are arranged with unequal central angles.

33. The device according to one of the claims 1 to 32, wherein the anchoring element (3.j) has a coaxial hollow (28) with a hole (20) that is also coaxial and penetrates the anchoring element (3.j) from the front end (5) to the back end (6).

34. The device according to one of the claims 1 to 26, wherein the anchoring element (3.j) has an anchoring segment (27) that has at least one blade (9) and abuts the back end (6) over a length (L) and at least the one blade (9) has a sawtooth surface structure, whereby the steep sides of the saw-teeth are oriented toward the back end (6) of the anchoring element (3.j).

35. The device according to claim 34, wherein the surface structure is attached on one side.

36. The device according to claim 34, wherein the surface structure is attached on both sides.

37. The device according to one of the claims 1 to 33, wherein the anchoring element (3.j) has an anchoring segment (27) that has at least one blade (9) and abuts the back end (6) over a length (L) and at least the one blade (9) has a fish-scale-like surface structure, whereby the steep sides of the scales are oriented toward the back end (6) of the anchoring element (3.j).

38. The device according to claim 37, wherein the surface structure is attached on one side.

39. The device according to claim 37, wherein the surface structure is attached on both sides.

40. The device according to one of the claims 15-30 or 33-39, wherein the anchoring segment (27), when viewed in cross-section section, has two blades (9) that lie in one plane and that are separated by a hollow (28) coaxially to the longitudinal axis (4), whereby the blades (9) have a thickness (D) of between 0.8 and 2 mm and a width (B) of between 2.5 mm and 4.5 mm and the hollow cylinder (28) has a diameter (d) of between 3 and 7 mm.

41. The device according to one of the claims 15 to 40, wherein the ratio of the width (B) to the thickness (D) is basically between 1 and 14.

42. The device according to claim 41, wherein the ratio of the width (B) to the thickness (D) is basically between 3 and 6.

43. The device according to one of the claims 15 to 42, wherein at least one blade (9) is designed in the shape of a spiral.

44. The device according to claim 43, wherein at least one blade (9) has a lead S of between 60 and 300 mm.

45. The device according to claim 44, wherein at least one blade (9) has a lead S of between 100 and 240 mm.

46. The device according to one of the claims 43 to 45, wherein the twisting angle $\alpha$ over length (L) of the blades (9) is between 0 and 360°.

47. The device according to claim 46, wherein the twisting angle $\alpha$ over length (L) of the blades (9) is between 0 and 180°.

48. The device according to claim 47, wherein the twisting angle $\alpha$ over length (L) of the blades (9) is between 0 and 45°.

49. The device according to claim 47, wherein the twisting angle $\alpha$ over length (L) of the blades (9) is between 45 and 90°.

50. The device according to one of the claims 15 to 49, wherein at least one blade (9) is twisted in the shape of a spiral and the twist runs clockwise.

51. The device according to one of the claims 15 to 49, wherein at least one blade (9) is twisted in the shape of a spiral and the twist runs counter-clockwise.

**Revendications**

1. Dispositif destiné à la fixation d'os entre eux, notamment de corps vertébraux, comprenant

    A) un support longitudinal (1) avec un axe central (2); et
    B) n éléments d'ancrage (3.i) (2 ≤ i ≤ n) avec des axes longitudinaux (4), chacun une extrémité avant (5) et une extrémité arrière (6),
    C) les axes longitudinaux (4) des éléments d'ancrage (3.i) se trouvant dans un angle de 65° à 115° par rapport à l'axe central (2) du support longitudinal (1); et
    D) au moins un des éléments d'ancrage (3.j) (1 ≤ j ≤ n) ayant la forme d'une lame, et
    E) au minimum l'élément d'ancrage (3.j) comprenant sur l'extrémité arrière (6) des moyens de réception (7) avec un logement (11) pour le support longitudinal (1), munis d'organes de blocage (8;34) destinés au blocage amovible de la liaison entre le support longitudinal (1) et l'élément d'ancrage (3.j), et

F) la liaison arrêtée interdisant tout mouvement relatif entre le support longitudinal (1) et l'élément d'ancrage (3.j) et absorbant les forces et les moments dans toutes les directions des trois axes d'un système de coordonnées tridimensionnel,

**caractérisé en ce que**

G) au minimum l'élément d'ancrage (3.j) comprend un dispositif de transport (15) destiné à introduire l'élément d'ancrage (3.j) dans un os, parallèlement à l'axe longitudinal (4).

2. Dispositif selon la revendication 1, **caractérisé en ce que** l'élément d'ancrage minimum (3.j) (1 ≤ j ≤ n) est en forme de lame sur sa partie contiguë à l'extrémité arrière (6) de l'élément d'ancrage (3.j).

3. Dispositif selon la revendication 1 ou 2, **caractérisé en ce que** les organes de blocage (8) peuvent être manipulés par l'extrémité arrière (6) de l'élément d'ancrage minimum (3.j).

4. Dispositif selon une des revendications 1 à 3, **caractérisé en ce que** le logement (11) est ouvert latéralement, de sorte que l'on peut introduire le support longitudinal (1) dans le logement (11) transversalement à l'axe longitudinal (4).

5. Dispositif selon une des revendications 1 à 3, **caractérisé en ce que** le logement (7) est ouvert sur l'extrémité arrière (6), de sorte que l'on peut introduire le support longitudinal (1) dans le logement (11) parallèlement à l'axe longitudinal (4).

6. Dispositif selon une des revendications 1 à 5, **caractérisé en ce que** le dispositif transporteur (15) est conçu comme une vis transporteuse (16).

7. Dispositif selon la revendication 6, **caractérisé en ce que** la vis transporteuse (16) comprend une pointe de vis (17), une tige de vis (18), un segment fileté (21) contigu à la pointe de vis (17) et des organes d'entraînement (19).

8. Dispositif selon la revendication 7, **caractérisé en ce que** les organes d'entraînement (19) peuvent être manipulés par l'extrémité arrière (6) de l'élément d'ancrage (3.j).

9. Dispositif selon la revendication 8, **caractérisé en ce qu'**au moins un élément d'ancrage (3.j) présente coaxialement un alésage traversant (20) et **en ce que** cet alésage (20) peut recevoir la vis transporteuse (16), de sorte que la pointe de vis (17) et le segment fileté (21) dépassent axialement de l'extrémité avant (5) de l'élément d'ancrage (3.j).

10. Dispositif selon la revendication 8, **caractérisé en ce qu'**au moins un élément d'ancrage (3.j) présente coaxialement un alésage traversant (20) et **en ce que** cet alésage (20) peut recevoir la vis transporteuse (16), le segment fileté (21) étant intégré à l'élément d'ancrage (3.j) et une partie de sa circonférence dépassant radialement de l'élément d'ancrage (3.i), perpendiculairement à l'axe longitudinal (4).

11. Dispositif selon une des revendications 1 à 10, **caractérisé en ce que** l'extrémité arrière (6) d'au moins un des éléments d'ancrage (3.j) présente un segment conique (23) coaxial.

12. Dispositif selon la revendication 11, **caractérisé en ce que** le segment conique (23) présente un alésage fileté (24) concentrique et ouvert axialement à l'extrémité.

13. Dispositif selon la revendication 11, **caractérisé en ce qu'**une goupille (25) concentrique munie d'un filetage mâle est contiguë axialement à l'extrémité du segment conique (23).

14. Dispositif selon une des revendications 1 à 10, **caractérisé en ce que** les récepteurs (7) comprennent une tête cylindrique (26) coaxiale contiguë à l'extrémité arrière (6) de l'élément d'ancrage (3.j).

15. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui présente une section en substance rectangulaire avec une épaisseur (D) et une largeur (B), la lame (9) comprenant un premier axe transversal (22) parallèle aux côtés longs de la section.

16. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui, vue de dessus, converge en direction de l'extrémité avant (5).

17. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui, vue de dessus, diverge en direction de l'extrémité avant (5).

18. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui, vue de dessus, converge en une pointe (14) sur l'extrémité avant (5).

19. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui, vue de dessus, est biseautée d'un côté sur l'extrémité avant (5).

20. Dispositif selon une des revendications 1 à 14, **caractérisé en ce que** l'élément d'ancrage (3.j) comprend, sur une longueur (L), un segment d'ancrage (27) contigu à l'extrémité arrière (6) avec au moins une lame (9) qui, vue de dessus, est convexe sur l'extrémité avant (5).

21. Dispositif selon une des revendications 1 à 20, **caractérisé en ce que** l'élément d'ancrage (3.j) comprend, sur une longueur (L), un segment d'ancrage (27) contigu à l'extrémité arrière (6) avec au moins une lame (9) qui, vue en coupe longitudinale, converge en pointe sur l'extrémité avant (5).

22. Dispositif selon une des revendications 1 à 20, **caractérisé en ce que** l'élément d'ancrage (3.j) comprend, sur une longueur (L), un segment d'ancrage (27) contigu à l'extrémité arrière (6) avec au moins une lame (9) qui, vue en coupe longitudinale, est chanfreinée d'un côté sur l'extrémité avant (5).

23. Dispositif selon une des revendications 1 à 22, **caractérisé en ce que** l'élément d'ancrage (3.j) comprend, sur une longueur (L), un segment d'ancrage (27) contigu à l'extrémité arrière (6) avec au moins une lame (9) qui, vue en coupe transversale, se termine en pointe sur au moins une face latérale (32).

24. Dispositif selon une des revendications 1 à 22, **caractérisé en ce que** l'élément d'ancrage (3.j) comprend, sur une longueur (L), un segment d'ancrage (27) contigu à l'extrémité arrière (6) avec au moins une lame (9) qui, vue en coupe transversale, est chanfreinée d'un côté sur au moins une face latérale (32).

25. Dispositif selon une des revendications 1 à 24, **caractérisé en ce que** l'élément d'ancrage (3.j) comprend, sur une longueur (L), un segment d'ancrage (27) contigu à l'extrémité arrière (6) avec au moins une lame (9) qui est vrillée sur la longueur (L) autour d'une arête contiguë à une face latérale (32).

26. Dispositif selon une des revendications 1 à 24, **caractérisé en ce que** l'élément d'ancrage (3.j) comprend, sur une longueur (L), un segment d'ancrage (27) contigu à l'extrémité arrière (6) avec au moins une lame (9) qui est vrillée sur la longueur (L) autour de l'axe longitudinal (4).

27. Dispositif selon une des revendications 1 à 26, **caractérisé en ce que** l'élément d'ancrage (3.j) comprend, sur une longueur (L), un segment d'ancrage (27) contigu à l'extrémité arrière (6) avec deux lames (9).

28. Dispositif selon la revendication 27, **caractérisé en ce que** les lames (9), vues en coupe transversale, se situent dans un même plan.

29. Dispositif selon la revendication 27 ou 28, **caractérisé en ce que** les lames (9) sont séparées par une tige parallèlement à l'axe longitudinal (4).

30. Dispositif selon la revendication 29, **caractérisé en ce que** la tige est un corps creux (28) perforé parallèlement à l'axe longitudinal (4).

31. Dispositif selon une des revendications 1 à 26, **caractérisé en ce que** l'élément d'ancrage (3.j) comprend, sur une longueur (L), un segment d'ancrage (27) contigu à l'extrémité arrière (6) avec trois lames (9) ou plus et **en ce que** le segment d'ancrage (27) présente une section en étoile.

32. Dispositif selon une des revendications 1 à 26, **caractérisé en ce que** l'élément d'ancrage (3.j) comprend, sur une longueur (L), un segment d'ancrage (27) contigu à l'extrémité arrière (6) avec trois lames (9) ou plus, les lames (9) étant disposées selon des angles au centre inégaux, vus en coupe transversale de l'élément d'ancrage (27).

33. Dispositif selon une des revendications 1 à 32, **caractérisé en ce que** l'élément d'ancrage (3.j) comprend un corps creux (28) coaxial avec un alésage (20) également coaxial et traversant l'élément d'ancrage (3.j) de l'extrémité avant (5) à l'extrémité arrière (6).

34. Dispositif selon une des revendications 1 à 33, **caractérisé en ce que** l'élément d'ancrage (3.j) comprend, sur une longueur (L), un segment d'ancrage (27) contigu à l'extrémité arrière (6) avec au moins une lame (9) et **en ce que** cette lame (9) présente une structure de surface en dents de scie, les faces raides des dents de scie étant orientées vers l'extrémité arrière (6) de l'élément d'ancrage (3.j).

35. Dispositif selon la revendication 34, **caractérisé en ce que** la structure de surface se trouve sur une face.

36. Dispositif selon la revendication 34, **caractérisé en**

**ce que** la structure de surface se trouve sur les deux faces.

37. Dispositif selon une des revendications 1 à 33, **caractérisé en ce que** l'élément d'ancrage (3.j) comprend, sur une longueur (L), un segment d'ancrage (27) contigu à l'extrémité arrière (6) avec au moins une lame (9) et **en ce que** cette lame (9) présente une structure de surface en écailles, les faces raides des écailles étant orientées vers l'extrémité arrière (6) de l'élément d'ancrage (3.j).

38. Dispositif selon la revendication 37, **caractérisé en ce que** la structure de surface se trouve sur une face.

39. Dispositif selon la revendication 37, **caractérisé en ce que** la structure de surface se trouve sur les deux faces.

40. Dispositif selon une des revendications 15 à 30 ou 33 à 39, **caractérisé en ce que**, vu en coupe transversale, le segment d'ancrage (27) comprend deux lames (9) situées dans un plan, séparées par un corps creux (28) coaxialement à l'axe longitudinal (4), les lames (9) ayant une épaisseur (D) comprise entre 0,8 mm et 2 mm, une largeur (B) comprise entre 2,5 mm et 4,5 mm et le cylindre creux (28) ayant un diamètre (d) compris entre 3 mm et 7 mm.

41. Dispositif selon une des revendications 15 à 40, **caractérisé en ce que** le rapport entre la largeur (B) et l'épaisseur (D) est compris en substance entre 1 et 14.

42. Dispositif selon la revendication 41, **caractérisé en ce que** le rapport entre la largeur (B) et l'épaisseur (D) est compris en substance entre 3 et 6.

43. Dispositif selon une des revendications 15 à 42, **caractérisé en ce qu'**au moins une lame (9) est hélicoïdale.

44. Dispositif selon la revendication 43, **caractérisé en ce que** la lame (9) minimum présente un pas S compris entre 60 mm et 300 mm.

45. Dispositif selon la revendication 44, **caractérisé en ce que** la lame (9) minimum présente un pas S compris entre 100 mm et 240 mm.

46. Dispositif selon une des revendications 43 à 45, **caractérisé en ce que** l'angle de torsion $\alpha$ sur la longueur (L) des lames (9) est compris entre 0° et 360°.

47. Dispositif selon la revendication 46, **caractérisé en ce que** l'angle de torsion $\alpha$ sur la longueur (L) des lames (9) est compris entre 0° et 180°.

48. Dispositif selon la revendication 47, **caractérisé en ce que** l'angle de torsion $\alpha$ sur la longueur (L) des lames (9) est compris entre 0° et 45°.

49. Dispositif selon la revendication 47, **caractérisé en ce que** l'angle de torsion $\alpha$ sur la longueur (L) des lames (9) est compris entre 45° et 90°.

50. Dispositif selon une des revendications 15 à 49, **caractérisé en ce qu'**au moins une lame (9) est hélicoïdale et **en ce que** la torsion suit le sens des aiguilles d'une montre.

51. Dispositif selon une des revendications 15 à 49, **caractérisé en ce qu'**au moins une lame (9) est hélicoïdale et **en ce que** la torsion suit le sens inverse des aiguilles d'une montre.

6

8

13

13

12

11

1

7

10

2

1

3

10

27

9

L

4

9

4

5

5

14

B

D

Fig. 1

Fig. 2

Fig. 3

Fig. 4

Fig. 7a

Fig. 7b

Fig. 7c

Fig. 7d

Fig. 7e

Fig. 5a

Fig. 5b

Fig. 5c

Fig. 5d

Fig. 5e

Fig. 5f

Fig. 5g

Fig. 6a

Fig. 6b

EP 1 339 335 B1

Fig. 8a

Fig. 9a

Fig. 8b

Fig. 9b

Fig. 11

Fig. 15

Fig. 10

Fig. 12

Fig. 13

Fig. 14a

Fig. 14b

Fig. 14c

Fig. 16a

Fig. 16b

Fig. 17a

Fig. 17b

EP 1 339 335 B1

Fig. 18

Fig. 19